# EUROPÄISCHE PATENTSCHRIFT

(11) **EP 0 623 328 B1**
(45) Veröffentlichungstag und Bekanntmachung des Hinweises auf die Patenterteilung: **15.07.1998**
(21) Anmeldenummer: 94106552.6
(22) Anmeldetag: 27.04.1994
(51) Int. Cl.: A61F 9/00, A61B 17/32

(54) **Augenchirurgische Einrichtung zum Zerkleinern und Entfernen des Linsenkerns aus dem Auge eines Lebewesens**
Surgical apparatus for pulverizing and removing the nucleus from the lens of an eye of a living creature
Appareil chirurgical pour pulvériser et enlever le noyau du cristallin d'un oeil d'un être vivant

(30) Priorität: 07.05.1993 CH 1470/93; 09.04.1994 CH 1042/94
(43) Veröffentlichungstag der Anmeldung: 09.11.1994
(73) Patentinhaber: GRIESHABER & CO. AG SCHAFFHAUSEN, CH-8203 Schaffhausen (CH)
(72) Erfinder: Stegmann, Robert, Prof. M.D., Pretoria 0181 (ZA); Demmerle, Rudolf, CH-8200 Schaffhausen (CH)
(74) Vertreter: Althoff, Gerhard

(56) Entgegenhaltungen:
- EP-A- 0 147 192
- EP-A- 0 315 290
- US-A- 3 937 222
- US-A- 3 990 453
- US-A- 4 749 376
- US-A- 4 857 046

## Beschreibung

Die Erfindung bezieht sich auf eine Vorrichtung zum Zerkleinern und Entfernen des Linsenkerns aus dem Auge eines Lebewesens, bestehend aus einem im wesentlichen als Handstück ausgebildeten Gehäuse und darin angeordneten Antriebsmitteln, einem an dem einen Ende des Gehäuses angeordneten Kopfstück mit einem Führungsrohr sowie einer koaxial darin angeordneten Rohrsonde, wobei das mit einem Zuführkanal versehene Führungsrohr an dem im Kopfstück angeordneten Ende mit einer Infusionsleitung verbunden und an dem anderen Ende mit mindestens einer Austrittsöffnung versehen ist und wobei die Rohrsonde an dem aus dem Führungsrohr herausragenden Ende eine Ansaugöffnung und ein um eine gemeinsame Längsachse rotierend antreibbares Schneidelement aufweist sowie an dem gegenüberliegend im Kopfstück angeordneten Ende mit einer Absaugöffnung versehen ist, welche zum Absaugen des überschüssigen Fluids zusammen mit den Linsenpartikeln über eine gegenüber dem Zuführkanal abgedichtete Kammer sowie über eine daran angeschlossene Saugleitung mit einer Saugpumpe verbunden ist.

Die Linse (Okular) ist ein Teil des optischen Augensystems eines Lebewesens. Die hauptsächliche Funktion der Linse liegt in der Einstellungsfähigkeit der Brechkraft, welche im wesentlichen mit Hilfe der Zonularfasern in Verbindung mit dem Ziliarmuskel erfolgt. Die Einstellung (Akkomodation), bei welcher im wesentlichen die ellipsenförmige Formgebung der Linse verändert wird, erfolgt dabei in Abhängigkeit von der Beschaffenheit (Flexibilität) des Linsenkerns.

Durch Eintrübung und/oder Verhärtung des Linsenkerns, welche auch als sogenannter grauer Star (Katarakt) bekannt ist, wird das Sehvermögen erheblich beeinträchtigt. Hierbei unterscheidet man im wesentlichen zwischen angeborenen sowie altersbedingten Katarakten, wobei die durch Stoffwechselreduktionen und zunehmende Sklerotisierung altersbedingt entstehenden Linsentrübungen beim Menschen etwa vom 50. bis 60. Lebensjahr an auftreten können. Die das Sehvermögen des Auges beeinträchtigende Eintrübung kann jedoch auch durch Verletzungen, zum Beispiel durch Schlageinwirkungen, oder aber auch durch Unfälle entstehen. Zum Entfernen von Katarakten ist allgemein die Methode der intrakapsulären Extraktion bekannt, bei welcher die getrübte Linse als eine Einheit entfernt und danach beispielsweise eine Kunstlinse implantiert wird.

Zum Entfernen eines Katarakts ist aus der US-A 3,990,453 eine Vorrichtung bekannt, welche ein Gehäuse, einen darin angeordneten Motor, ein mit gleicher Achsrichtung am Gehäuse angeschraubtes Zwischenstück, ein daran mit gleicher Achsrichtung angeschraubtes Kopfstück sowie ein darin gelagertes Schneidwerkzeug umfasst, welches ein am vorderen freien Ende zum Zerkleinern des Linsenkerns mit einem Schneidkopf versehenes Innenrohr sowie ein darüber geschobenes und damit fest verbundenes Aussenrohr aufweist, wobei das Innenrohr sowie das Aussenrohr zusammen um eine gemeinsame Längsachse ausschliesslich rotierend angetrieben sind. Eine Relativbewegung des Innenrohres in bezug auf das Aussenrohr ist bei dieser Vorrichtung nicht vorgesehen und mit dem beispielsweise in Form einer angeschrägten oder sägezahnförmigen Schneidkante ausgebildeten Schneidkopf können die Linsenkerne, insbesondere aber verhältnismässig harte Linsenkerne nur unzureichend zerkleinert werden ohne dabei die Innenwand des Kapselsacks zu verletzen.

Aus der US-A 4,320,761 ist eine weitere Vorrichtung zum Entfernen eines Katarakts bekannt, welche ein Gehäuse, ein daran befestigtes Kopfstück, eine darin angeordnete erste Hohlnadel sowie eine in achsparallelem Abstand aussenseitig an der ersten Hohlnadel befestigte zweite Hohlnadel umfasst, wobei koaxial in der ersten Hohlnadel eine rotierend angetriebene Welle angeordnet ist, welche an dem freien Ende ein verdrilltes Schneidelement aufweist, mittels welchem der zu beseitigende Graue Star unter gleichzeitiger Zufuhr eines Spülfluids zerkleinert und über die Hohlnadel abgesaugt wird.

Aus der DE-A 40 80 594 ist ein augenchirurgisches Instrument zum Zerkleinern und Absaugen von Linsentrümmern bekannt, welches im wesentlichen ein Gehäuse, ein mit Antriebsmitteln in Wirkverbindung stehendes Aufnahmeteil sowie eine in Form einer Hohlnadel ausgebildete und ausschliesslich mit hoher Frequenz in longitudinaler Richtung bewegbare Ultraschallsonde umfasst, mittels welcher unter Zufuhr einer Spülflüssigkeit die zerkleinerten Linsentrümmer abgesaugt werden.

Zum Zerkleinern eines Linsenkerns sowie zum Absaugen der dabei entstehenden Linsenkerntrümmer sind aus den Druckschriften (US-A 3,976,077 und US-A 4,002,169) weitere mit entsprechend ausgebildeten Schneidelementen versehene Instrumente bekannt.

Weiterhin ist aus der EP-A 0 147 192 eine Vorrichtung bekannt, welche ein an einem flexiblen Katheter angeordnetes Kopfstück sowie einen daran angeordneten und rotierend antreibbaren Schneidkopf aufweist, welcher an der Stirnseite mit in Umfangsrichtung verteilt angeordneten und jeweils mit einer sich konisch verjüngenden oder kreisbogenförmigen Schneidkante versehene Schneidelemente zum Enfernen von Ablagerungen aufweist.

Der Erfindung liegt die Aufgabe zugrunde, eine Vorrichtung gemäss der im Oberbegriff des Anspruchs 1 genannten Gattung dahingehend zu verbessern, dass der getrübte Linsenkern zerkleinert und emulsifiziert sowie im wesentlichen als breiige Masse vollständig entfernt werden kann, ohne dass dabei die hochempfindliche Innenwand des Kapselsacks verletzt wird.

Gelöst wird diese Aufgabe gemäss der Erfindung dadurch, dass die Rohrsonde mit dem daran angeordneten Schneidelement relativ zu dem feststehend in dem Kopfstück gelagerten Führungsrohr zusätzlich zu der Rotationsbewegung in axialer Richtung der Längsachse oszillierend antreibbar ist und dass dass Schneidelement mit mindestens zwei im wesentlichen in einer die Längsachse beinhaltende Ebene liegende Flügelstücken versehen ist.

Durch die angegebenen Massnahmen besteht nunmehr die Möglichkeit, mit der oszilierenden Bewegung des Schneidelements verhältnismässig harte Linsenkerne zu zerkleinern sowie mit der rotierenden Bewegung des Schneidelements die Epithelzellen an der vorderen Innenseite des Kapselsacks zu entfernen, ohne diesen dabei zu verletzen. Als besonders vorteilhaft hat sich dabei die kombinierte Rotations- und hochfrequente Oszillationsbewegung erwiesen.

Weitere Merkmale der Erfindung ergeben sich aus der folgenden Beschreibung in Verbindung mit der Zeichnung und den einzelnen Patentansprüchen.

Ausführungsbeispiele der Erfindung werden nachstehend anhand der Zeichnung beschrieben. Es zeigt:
**Fig.1** den in grösserem Massstab und im Schnitt dargestellten vorderen Augenabschnitt eines Lebewesens sowie eine augenchirurgische Vorrichtung zum Entfernen des Linsenkerns;
**Fig.2** die schematisch dargestellte und mit einem Schneidwerkzeug versehene augenchirurgische Vorrichtung mit zugeordneter Saugpumpe und Infusionseinheit;
**Fig.3** die zur Aufnahme des Schneidwerkzeuges ausgebildete und in grösserem Massstab sowie in Ansicht dargestellte augenchirurgische Vorrichtung gemäss Fig. 2;
**Fig.4** ein in grösserem Massstab sowie im Schnitt dargestelltes und mit dem Schneidwerkzeug versehenes Kopfstück für die augenchirurgische Vorrichtung gemäss Fig.3;
**Fig.5** als erstes Ausführungsbeispiel ein in grösserem Massstab und räumlich dargestelltes Teilstück des Schneidwerkzeuges mit daran angeordneten Schneidelementen;
**Fig.6** das im Schnitt dargestellte Teilstück des Schneidwerkzeuges gemäss Fig. 5;
**Fig.7** das in Seitenansicht dargestellte Teilstück des Schneidwerkzeuges gemäss Fig.6;
**Fig.8** als zweites Ausführungsbeispiel ein in grösserem Massstab und im Schnitt dargestelltes Teilstück des Schneidwerkzeuges mit daran angeordneten Schneidelementen;
**Fig.9** das in Seitenansicht dargestellte Teilstück des Schneidwerkzeuges gemäss Fig.8;
**Fig.10** das in Draufsicht dargestellte Schneidwerkzeug gemäss Fig.8;
**Fig.11** als drittes Ausführungsbeispiel ein in grösserem Massstab und räumlich dargestelltes Teilstück des Schneidwerkzeuges mit daran angeordneten Schneidelementen;
**Fig.12** das in Draufsicht dargestellte Schneidwerkzeug gemäss Fig.11;
**Fig.13** das im Schnitt dargestellte Teilstück des Schneidwerkzeuges gemäss Fig.11; und
**Fig.14** eine weitere, im Schnitt sowie als Teilstück dargestellte Variante des Schneidwerkzeuges gemäss Fig.11.

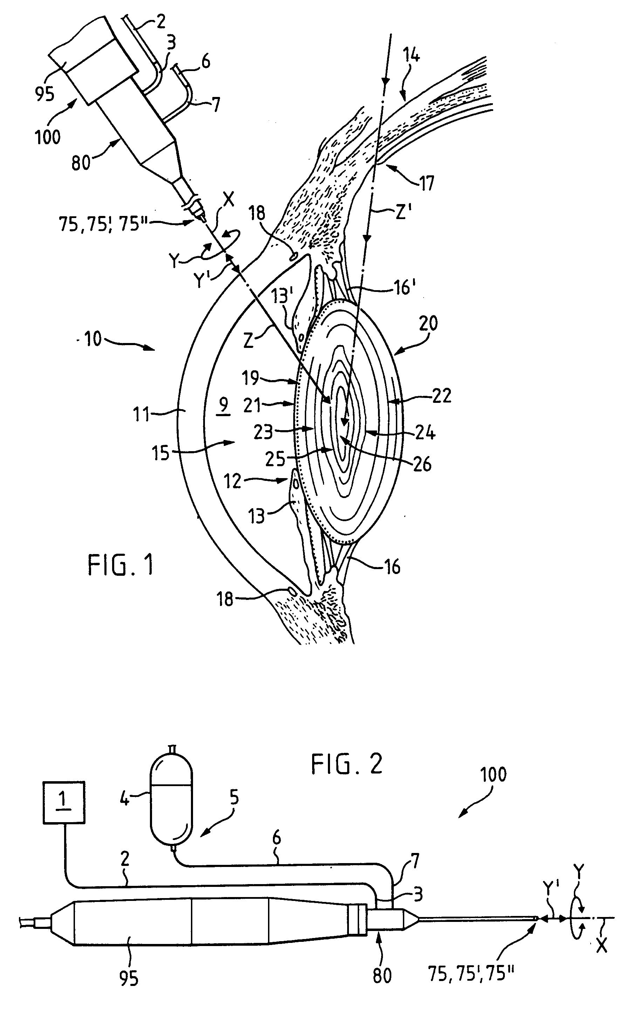

In Fig.1 ist ein in der Gesamtheit mit 10 bezeichneter Augenabschnitt eines Lebewesens in grösserem Massstab und im Schnitt dargestellt und man erkennt die Hornhaut 11 (Cornea), die Vorderkammer 9, die als Gesamtheit mit 12 bezeichnete Regenbogenhaut (Iris) mit den beiden zirkulären Bereichen 13 und 13', die Lederhaut 14 (Sklera), die Pupille 15, die Linse 20 (Okular) mit den Strahlenbändern 16,16' (Zonularfasern) sowie die gesamthaft mit 17 bezeichnete Pars plana und den mit 18 bezeichneten zirkulären Schlemmschen Kanal.

Die als histologischer Schnitt dargestellte Linse 20 besteht aus mehreren, schematisch dargestellten Diskontinuitätszonen, welche sich, von aussen nach innen gesehen, wie folgt zusammensetzen: **a)** vordere und hintere Kapselhaut 19, **b)** nur vorne bis etwa zum Äquator reichende Epithelzellen 21 der Linse, **c)** vordere und hintere Abspaltungsfläche 22, **d)** vordere und hintere Alterskernzone 23, **e)** vordere und hintere äussere Embryonalkernzone 24, **f)** vordere und hintere innere Embryonalkernzone 25 und **g)** zentraler Interval 26.

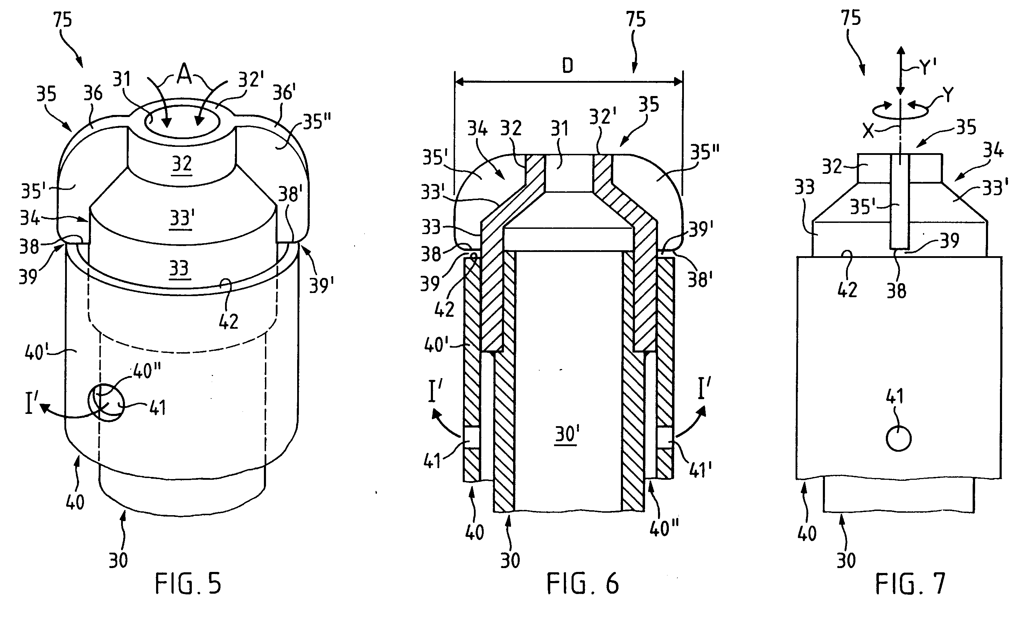

Weiterhin erkennt man in Fig.1 ein Teilstück einer schematisch dargestellten augenchirurgischen Vorrichtung 100, welche im wesentlichen ein als Griffstück ausgebildetes Gehäuse 95 und ein daran befestigtes Kopfstück 80 umfasst, welches zur Aufnahme eines in Fig.1 schematisch dargestellten Schneidwerkzeuges 75 oder 75' beziehungsweise 75'' ausgebildet ist. Das in dem Kopfstück 80 angeordnete Schneidwerkzeug 75;75';75'' steht mit nicht dargestellten, beispielsweise im Gehäuse 95 angeordneten Antriebsmitteln derart in Wirkverbindung, dass ein daran angeordnetes Schneidelement 35,55 oder 65 (Fig.5 bis Fig.14) um eine Längsachse X in Pfeilrichtung Y rotierend sowie in Richtung der Längsachse X gemäss Pfeilrichtung Y' oszillierend angetrieben werden kann. Die nicht dargestellten Antriebsmittel sind vorzugsweise so ausgebildet, dass eine sogenannte Überlagerung der genannten Bewegungen und somit eine Kombination der oszillierenden und rotierenden Bewegungen möglich ist.

Zum Entfernen eines getrübten Linsengewebes oder Linsenkerns (Katarakt) wird in einer ersten Phase die Regenbogenhaut 12 entweder medikamentös oder durch entsprechend hakenförmig ausgebildete, nicht dargestellte Instrumente (Retractors) erweitert und in die Hornhaut 11 eine im wesentlichen schlitzförmige Öffnung (nicht dargestellt) eingeschnitten. Anschliessend wird die Vorrichtung 100 mit dem entsprechenden Schneidwerkzeug 75, 75' oder 75'', wie in Fig.1 schematisch mit Pfeilrichtung Z dargestellt, dem Augenabschnitt 10 zugeführt und in das Auge eingeführt. Hierbei wird ein Schneidelement 35, 55 oder 65, welches an einer koaxial in einem Führungsrohr 40, 45 oder 70 angeordneten Rohrsonde 30,50 oder 60 angeordnet und befestigt ist, durch die in der Hornhaut 11 und in der Kapselhaut 19 vorgesehene Öffnung (nicht dargestellt) in die Linse 20 eingeführt. Mittels dem rotierend und/oder oszillierend angetriebenen Schneidelement 35 oder 55 beziehungsweise 65 (Fig.5 bis Fig.14) kann nunmehr der Linsenkern in Partikel zerkleinert und gleichzeitig in Form einer breiigen Masse abgesaugt (Aspiration) und aus dem Kapselsack entfernt werden.

Wie in Fig.1 weiterhin schematisch dargestellt, kann die Vorrichtung 100 jedoch auch in Pfeilrichtung Z' durch die Pars plana 17 in die Linse 20 eingeführt werden.

Fig.2 zeigt die in schematischer Ansicht dargestellte augenchirurgische Vorrichtung 100 mit dem als Handgriff ausgebildeten Gehäuse 95 und dem daran angeordneten Kopfstück 80. Die Vorrichtung 100 ist einerseits über eine Saugleitung 2 an eine Saugpumpe 1 angeschlossen und steht andererseits über eine Infusionsleitung 6 mit einem Infusionsbehälter 4 einer schematisch dargestellten Infusionseinheit 5 in Verbindung. Die Saugleitung 2 und Infusionsleitung 6 sind an entsprechend im Abstand zueinander an dem Kopfstück 80 angeordnete Stutzen 3 beziehungsweise 7 angeschlossen. Weiterhin erkennt man in Fig.2 das schematisch dargestellte und am Kopfstück 80 beispielsweise auswechselbar angeordnete Schneidwerkzeug 75 oder 75' beziehungsweise 75''.

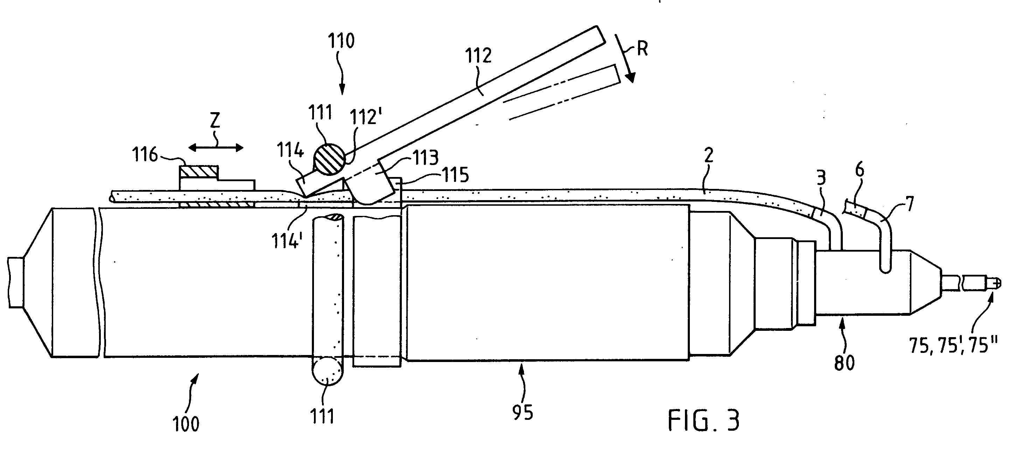

In Fig.3 ist in grösserem Massstab und in Ansicht die augenchirurgische Vorrichtung 100 dargestellt und man erkennt das beispielsweise als Zylinderkörper ausgebildete Gehäuse 95 mit dem an dem einen Ende angeordneten Kopfstück 80 und dem darin gelagerten Schneidwerkzeug 75 oder 75' beziehungsweise 75''. An dem Kopfstück 80 ist der Stutzen 3 für die Saugleitung 2 und im Abstand dazu der Stutzen 7 für die Infusionsleitung 6 angeordnet und mit nicht dargestellten Mitteln befestigt.

An dem Gehäuse 95 ist weiterhin eine schematisch dargestellte Klemmvorrichtung 110 angeordnet, welche im wesentlichen eine am Gehäuse 95 angeordnete Halterung 115 umfasst. In der Halterung 115 ist in nicht näher dargestellter Weise ein mit einem Lagerstück 113 und mit einem Druckstück 114 versehener und gegen die Rückstellkraft eines Federelements schwenkbarer Hebelarm 112 gelagert. Im dargestellten Ausführungsbeispiel ist der Hebelarm 112 mit einer kreisbogenförmigen Ausnehmung 112' versehen, in welche beispielsweise ein O-Ring 111 oder ein funktionell analoges Federelement eingelegt ist. Der O-Ring 111 hat eine relativ hohe federelastische Rückstellkraft. Durch in Pfeilrichtung R auf den Hebelarm 112 orientierten Druck wird dieser verschwenkt, derart, dass das Druckstück 114 ausser Eingriff der Saugleitung 2 gelangt. Durch Loslassen des Hebelarms 112 wird die Saugleitung 2 durch das Druckstück 114 beispielsweise gegen ein schematisch dargestelltes Auflageteil 114' gedrückt und dabei abrupt abgeklemmt (Zufuhr des Fluids ist unterbrochen).

In diesem Zustand wird die strömungsmässig unterbundene Saugleitung 2 in bezug auf die Saugpumpe 1 hinter dem Druckstück 114 entleert und somit bei laufender Saugpumpe 1 ein Vakuum aufgebaut, mittels welchem bei Betätigung des Hebelarms 112 in Pfeilrichtung R an der Spitze (Ansaugöffnung) des Schneidelements 35 oder 55 beziehungsweise 65 eine etwa schlagartig wirkende Saugkraft erzeugt wird, durch welch auch grössere Linsenkernstücke oder zusammenhängende Gewebeteilchen zuverlässig entfernt werden können.

An dem Gehäuse 95 ist im Abstand zu der Halterung 115 ein Fixierelement 116 vorgesehen, welches beispielsweise in nicht näher dargestellter Weise in Doppelpfeilrichtung Z verschiebbar am Gehäuse 95 geführt ist. Bei einer nicht näher dargestellten Variante besteht jedoch auch die Möglichkeit, das in Doppelpfeilrichtung Z verstellbare Fixierelement 116 an der Saugleitung 2 anzuordnen. Das Fixierelement 116 ist derart ausgebildet, dass der Hebelarm 112 in der in Pfeilrichtung R verschwenkten Stellung (der Durchfluss durch die Saugleitung 2 ist frei) festgehalten wird und erst durch Verschieben des Fixierelements 116 wieder freigegeben wird.

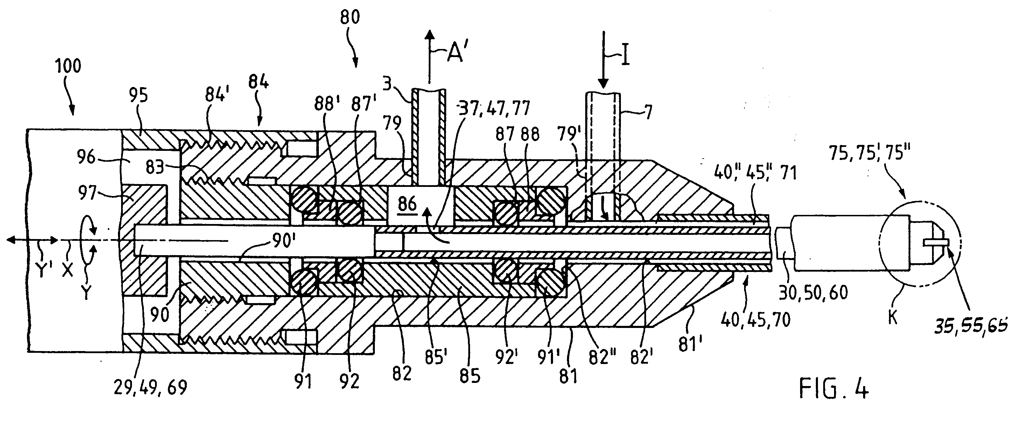

In Fig.4 ist in grösserem Massstab und im Schnitt das Kopfstück 80 für die augenchirurgische Vorrichtung 100 gemäss Fig.2/3/ dargestellt. Das an dem einen Ende mit einem zylindrischen Teilstück 81 und einem angeformten konischen Teilstück 81' versehene Kopfstück 80 hat einen im wesentlichen als Sacklochbohrung ausgebildeten und durch eine Stirnwand 82'' begrenzten Innenraum 82 sowie einen Kanal 82', welcher mit dem zylindrischen Innenraum 82 in Verbindung steht.

In dem zylindrischen Teilstück 81 des Kopfstücks 80 sind zwei in axialer Richtung im Abstand zueinander angeordnete und zur Aufnahme und Befestigung der Stutzen 3 und 7 ausgebildete Bohrungen 79 und 79' vorgesehen. Das andere Ende des Kopfstücks 80 ist beispielsweise als Schraubstück 84 ausgebildet und mit einem Aussengewinde 84' sowie mit einem Innengewinde 83 versehen. Das Schraubstück 84 dient zur aufschraubbaren Befestigung des als Hohlzylinderkörper ausgebildeten Gehäuses 95. In dem Innenraum 82 des Kopfstücks 80 ist ein zylindrisches Zwischenstück 85 angeordnet. Das Zwischenstück 85 wird von einer in axialer Richtung orientierten Bohrung 85' durchdrungen und ist weiterhin mit einer Saugkammer 86 und an den Enden jeweils mit einer abgesetzt ausgebildeten Ausnehmung 87,87' versehen. In den beiden abgesetzten Ausnehmungen 87,87' ist jeweils eine O-Ringdichtung 92,92' angeordnet und durch einen zugeordneten Stützring 88,88' gehalten. Der einzelne im Profilquerschnitt L-förmig ausgebildete Stützring 88,88' dient zur Aufnahme einer weiteren O-Ringdichtung 91,91'. Mittels einer in das Innengewinde 83 des Schraubstücks 84 einschraubbaren und mit einer Durchgangsbohrung 90' versehenen Schraubbuchse 90 werden die Stützringe 88,88' sowie das Zwischenstück 85 mit den zugeordneten O-Ringdichtungen 91,92 und 91',92' relativ zu der Stirnwand 82'' des Innenraumes 82 im Kopfstück 80 gegeneinander verspannt.

Wie in Fig.4 dargestellt, steht die im zylindrischen Teilstück 81 des Kopfstücks 80 vorgesehene Bohrung 79 für den Aspirationsanschluss A' über die im Zwischenstück 85 angeordnete Saugkammer 86 sowie über mindestens eine in dem jeweiligen Endstück der um die Längsachse X rotierend angetriebenen Rohrsonde 30;50;60 angeordnete Durchtrittsöffnung 37;47;77 mit der an dem jeweiligen schneidelement 35;55;65 Vorgesehenen Ansaugöffnung (Fig.5 bis 11) in Verbindung. Die in der Rohrsonde 30;50;60 angeordnete/n Durchtrittsöffnung/en 37;47;77 sind von der Saugkammer 86 zirkulär umgeben, so dass die Saugwirkung bei der Rotations- und/oder Longitudinal bewegung der Rohrsonde 30;50;60 nicht unterbrochen wird und die Linsenpartikel und Gewebeteilchen kontinuierlich von der Saugpumpe 1 abgesaugt werden.

Die ebenfalls im Teilstück 81 angeordnete Bohrung 79' für den Infusionsanschluss I steht über den zwischen der Aussenwand der Rohrsonde 30;50;60 und der Innenwand des Führungsrohres 40;45;70 vorgesehenen, kreisringförmigen Zuführkanal 40'',45'', 71 mit den im Bereich des Schneidelements 35;55;65 am Fuhrungsrohr 40;45;70 vorgesehenen Austrittsöffnungen (Fig.5 bis 14) in Verbindung. Der Zuführkanal 40'',45'',71 ist gegenüber der Saugkammer 86 durch den O-Ring 91' abgedichtet.

Weiterhin erkennt man in Fig.4 das teilweise im Schnitt beziehungsweise in Ansicht dargestellte und auswechselbar in das Kopfstück 80 einsetzbare Schneidwerkzeug 75 oder 75' beziehungsweise 75'' mit den am vorderen Ende angeordneten Schneidelementen 35;55;65. Das einzelne Schneidwerkzeug 75;75';75'' ist mit einem äusseren Führungsrohr 40 oder 45 beziehungsweise 70 im vorderen, konischen Teilstück 81' des Kopfstücks 80 mit nicht dargestellten Mitteln befestigt, vorzugsweise aber lösbar befestigt.

Das Führungsrohr 40 wird von der in axialer Richtung orientierten und als Hohlnadel ausgebildeten Rohrsonde 30 durchdrungen. An dem einen dem Schneidelemen 35;55;65 gegenuberliegenden Ende ist, wie in Fig.4 schematisch dargestellt, an der Rohrsonde 30;50;60 jeweils die Antriebswelle 29;49;69 angeordnet und mit nicht dargestellten Mitteln befestigt. Die jeweilige Antriebswelle 29;49;69 ist mit nicht dargestellten Mitteln mit einem Kupplungsstück 97 wirkverbunden. Mittels im Innenraum 96 des Gehäuses 95 angeordneter und mit dem Kupplungsstück 97 wirkverbundener Antriebsmittel (nicht dargestellt) wird die Antriebswelle 29;49;69 sowie die damit wirkverbundene Rohrsonde 30;50;60 zusammen mit dem Schneidelement 35;55; 65 relativ zu dem feststehenden Führungsrohr 40;45;70 um die Längsachse X in Pfeilrichtung Y rotierend angetrieben und in axialer Richtung gemäss Doppelpfeilrichtung Y' mit einer Frequenz in der Grössenordnung von 200 bis 1000 Hz in longitudinaler Richtung bewegt.

An dieser Stelle wird darauf hingewiesen, dass die Schneidwerkzeuge 75' beziehungsweise 75'' mit den einzelnen Teilen weitgehend analog dem vorstehend beschriebenen Ausführungsbeispiel ausgebildet sind. Abweichungen der einzelnen Schneidwerkzeuge 75 oder 75' beziehungsweise 75'' bestehen im wesentlichen in der Ausbildung und Anordnung der einzelnen Schneidelemente 35 oder 55 beziehungsweise 65. Die Schneidwerkzeuge 75 oder 75 beziehungsweise 75'' werden nachstehend in Verbindung mit den Figuren 5 bis 13 im einzelnen beschrieben.

Die Figuren 5 bis 7 zeigen als erstes Ausführungsbeispiel das in Fig.4 durch einen Kreis K bezeichnete Teilstück des Schneidwerkzeuges 75 in grösserem Massstab, wobei in Fig.5 das Schneidwerkzeug in räumlicher Darstellung, in Fig.6 im Schnitt und in Fig.7 in Seitenansicht dargestellt ist. Das Schneidwerkzeug 75 umfasst im wesentlichen die koaxial im Führungsrohr 40 angeordnete innere Rohrsonde 30, wobei zwischen der inneren Rohrsonde 30 und dem Führungsrohr 40, wie in Fig.6 dargestellt, der kreisringförmige Zufuhrkanal 40'' vorgesehen ist.

An der inneren Rohrsonde 30 ist ein im wesentlichen trichterförmig ausgebildeter und mit einer Ansaugöffnung 31 versehener Haltekörper 34 angeordnet, welcher ein erstes zylindrisches Teilstück 33, ein daran angeformtes konisches Teilstück 33' sowie ein daran angeformtes zweites zylindrisches Teilstück 32 umfasst. Der Haltekörper 34 ist mit dem ersten zylindrischen Teilstück 33 auf ein abgesetzt ausgebildetes Teilstück (nicht bezeichnet) der inneren Rohrsonde 30 aufgesteckt und beispielsweise durch eine Schweissverbindung (Laserschweissung) befestigt (Fig. 6). An den aus dem Führungsrohr 40 herausragenden Teilstücken 33,33' und 32 des Haltekörpers 34 sind mindestens zwei diametral gegenüberliegende Flügelstücke 35' und 35'' angeordnet. In dem Haltekörper 34 sind analog der Flügelstücke mindestens zwei diametral gegenüberliegend angeordnete Schlitze (nicht bezeichnet) vorgesehen, in welchen die beiden Flügelstücke 35' ,35'' angeordnet und in nicht näher dargestellter Weise, beispielsweise durch Laserschweissung befestigt sind. Die beiden Flügelstücke 35' und 35'' sind je mit einer etwa kreisbogenförmig abgerundeten Schneidkante 36,36' (Fig.5) versehen und bilden zusammen das in der Gesamtheit mit 35 bezeichnete Schneidelement.

Bei diesem Ausführungsbeispiel bilden die beiden Flügelstücke 35' und 35'' mit der oberen kreisringförmigen Fläche 32' des zweiten zylindrischen Teilstücks 32 eine ebene Fläche, an welche sich die abgerundeten und schematisch dargestellten Schneidkanten 36 und 36' anschliessen. Zwischen der ringförmigen Oberkante 42 des Führungsrohres 40 und den Unterkanten 38,38' der beiden Flügelstücke 35',35'' ist jeweils ein Spalt 39,39' vorgesehen.

Der in radialer Richtung über die beiden Flügelstücke 35' ,35'' gemessene Abstand D (Aussenmass des Schneidelements 35) liegt etwa in der Grössenordnung von 1,6 mm und ist grösser als der in der Grössenordnung von 1,4 mm bis 1,5 mm liegende äussere Durchmesser (nicht bezeichnet) des Fuhrungsrohres 40.

Fig.6 zeigt das in grösserem Massstab und im Schnitt dargestellte Schneidwerkzeug 75 und man erkennt das Führungsrohr 40, den koaxial darin angeordneten und an der inneren Rohrsonde 30 befestigten Haltekörper 34 sowie das daran angeordnete und befestigte Schneidelement 35. In dem Führungsrohr 40 sind in axialer Richtung im Abstand zu der ringförmigen Oberkante 42 mindestens zwei diametral gegenüberliegende und die Wand 40' durchdringende Austrittsöffnungen 41 und 41' vorgesehen, welche mit dem zwischen der inneren Rohrsonde 30 und dem Führungsrohr 40 bestehenden, kreisringförmigen Zuführkanal 40'' in Verbindung stehen.

In Fig.7 ist das Schneidwerkzeug 75 in Ansicht dargestellt und man erkennt das Führungsrohr 40 mit der einen Austrittsöffnung 41, die koaxial im Führungsrohr 40 angeordnete innere Rohrsonde 30 sowie den mit dem Schneidelement 35 versehenen Haltekörper 34. Die Rohrsonde 30 sowie der Haltekörper 34 mit dem Schneidelement 35 sind, wie bereits in Verbindung mit Fig.2 erwähnt, mittels der im Gehäuse 95 (Fig.3,4) angeordneten Antriebsmittel einerseits um eine gemeinsame Achse X in Pfeilrichtung Y relativ zum feststehenden Führungsrohr 40 drehbar und andererseits in axialer Richtung gemäss Pfeilrichtung Y' relativ zum Führungsrohr 40 bewegbar.

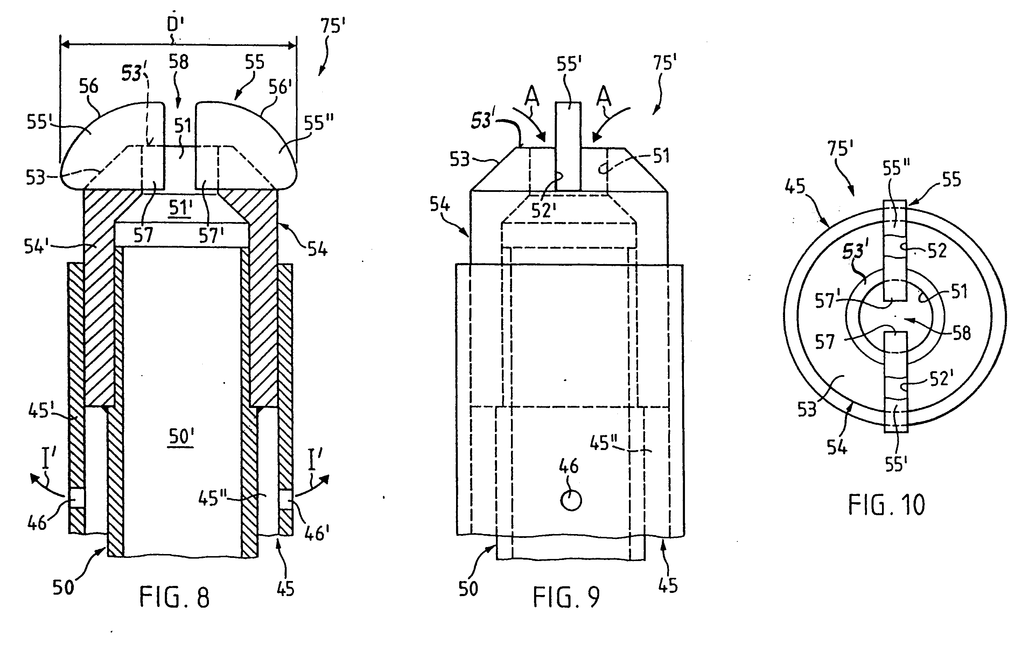

Die Figuren 8 bis 10 zeigen als zweites Ausführungsbeispiel das in Fig.4 durch den Kreis K bezeichnete Teilstück des Schneidwerkzeuges 75' in grösserem Massstab. In Fig.8 ist das Schneidwerkzeug im Schnitt, in Fig.9 in Seitenansicht und in Fig.10 in Draufsicht dargestellt. Das Schneidwerkzeug 75' umfasst das mit zwei Austrittsöffnungen 46,46' versehene Führungsrohr 45, die koaxial darin angeordnete Rohrsonde 50, einen Haltekörper 54 sowie das daran angeordnete Schneidelement 55. Die beiden die Wandung 45' des Führungsrohres 45 durchdringenden Öffnungen 46,46' stehen mit dem zwischen dem Führungsrohr 45 und der Rohrsonde 50 vorgesehenen kreisringförmigen Zufuhrkanal 45'' in Verbindung.

Das Schneidwerkzeug 75' ist im wesentlichen analog dem vorstehend in Verbindung mit den Figuren 5 bis 7 beschriebenen Schneidwerkzeug 75 ausgebildet. Der zur Aufnahme und Befestigung des Schneidelements 55 ausgebildete Haltekörper 54 ist mit einem ersten, zylindrischen Teilstück 54' auf ein abgesetzt ausgebildetes Teilstück (nicht bezeichnet) der inneren Rohrsonde 50 aufgesteckt und durch eine Laserschweissung befestigt.

Abweichend von dem ersten Schneidwerkzeug 75 gemäss Fig.5 bis Fig.7 ist bei dem Schneidwerkzeug 75' gemäss Fig.8 bis Fig.10 an dem mit einer Ansaugöffnung 51 und einem daran anschliessenden Innenraum 51' versehenen Haltekörper 54 ein konisches Teilstück 53 angeformt. In dem konischen Teilstück 53 sind mindestens zwei diametral gegenüberliegend angeordnete und in axialer Richtung des Haltekörpers 54 orientierte Schlitze 52,52' (Fig.10) vorgesehen, in welchen die beiden Flügelstücke 55' und 55'' des Schneidelements 55 angeordnet und in nicht näher dargestellter Weise befestigt sind. Die beiden Flügelstücke 55' ,55'' ragen, wie in Fig.8 und Fig.10 dargestellt, jeweils mit einem Teilstück 57,57' bis in die Ansaugöffnung 51 und sind durch einen Spalt 58 beabstandet. Die beiden Flügelstücke 55' und 55'' sind weiterhin mit einer etwa kreisbogenförmig abgerundeten, schematisch dargestellten Schneidkante 56, 56' versehen, welche zusammen das in der Gesamtheit mit 55 bezeichnete Schneidelement bilden. Die Flügelstücke 55' und 55'' sind weiterhin mit den Schneidkanten 56 und 56' in axialer Richtung mit geringem Abstand über die zirkuläre Fläche 53' des konischen Teilstücks 53 angeordnet.

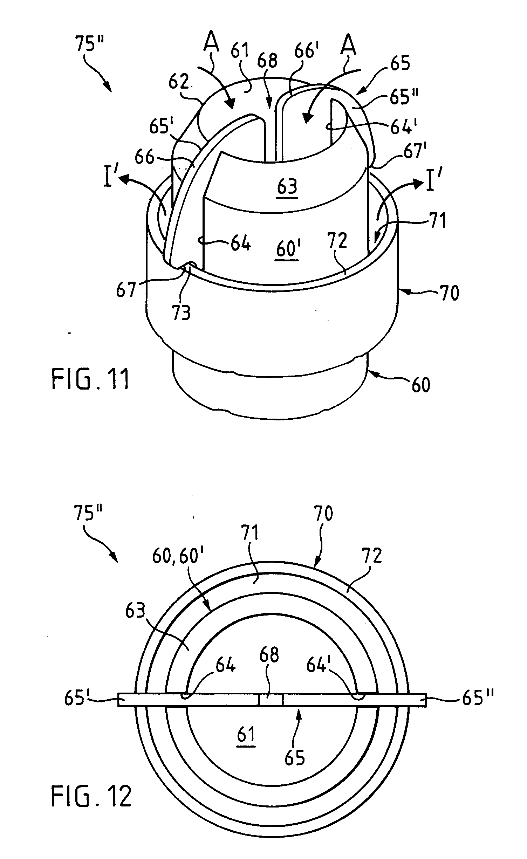

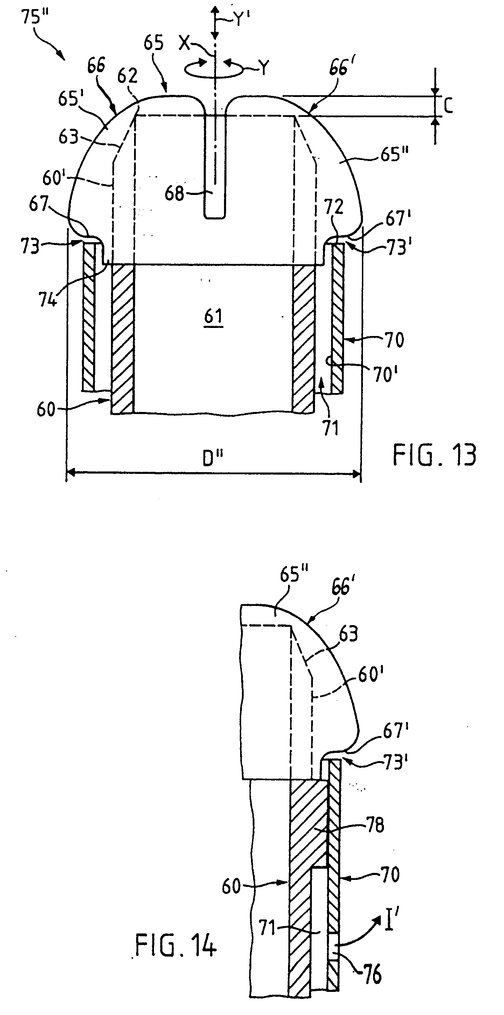

Die Figuren 11 bis 13 zeigen als drittes Ausführungsbeispiel das in Fig.4 durch den Kreis K bezeichnete Schneidwerkzeug 75'' in grösserem Massstab. In Fig.11 ist das Schneidwerkzeug 75'' in räumlicher Darstellung, in Fig.12 in Draufsicht und in Fig.13 im Schnitt dargestellt. Das Schneidwerkzeug 75'' umfasst im wesentlichen die im Führungsrohr 70 koaxial angeordnete innere Rohrsonde 60, wobei zwischen der inneren Rohrsonde 60 und dem Führungsrohr 70 der kreisringförmige Zuführkanal 71 vorgesehen ist.

Abweichend von den beiden ersten Ausführungsbeispielen gemäss der Figuren 5 bis 10 ist bei dem Schneidwerkzeug 75'' gemäss Fig.11 bis 13 die in axialer Richtung mit einem Teilstück 60' aus dem Führungsrohr 70 herausragende innere Rohrsonde 60 mit einer konisch angeschrägten Schneidfläche 63 versehen, welche an dem herausragenden Ende im wesentlichen als kreisringförmige Schneidspitze 62 ausgebildet ist. In dem herausragenden Teilstück 60' der inneren Rohrsonde 60 sind mindestens zwei diametral gegenüberliegend angeordnete und in axialer Richtung orientierte Schlitze 64,64' vorgesehen. In den Schlitzen 64,64' ist das Schneidelement 65 angeordnet und in nicht dargestellter Weise, vorzugsweise durch Laserschweissung, an der inneren Rohrsonde 60 befestigt.

Das Schneidelement 65 ist durch einen in axialer Richtung der Rohrsonde 60 orientierten Schlitz 68 teilweise in zwei Flügelstücke 65' und 65'' unterteilt. Die beiden Flügelstücke 65' ,65'' sind jeweils mit einer schematisch dargestellten Schneidkante 66,66' versehen. Die an den beiden Flügelstücken 65',65'' angeordneten Schneidkanten 66,66' reichen, wie in Fig.13 dargestellt, in axialer Richtung mit geringem Abstand C über die zirkuläre Schneidspitze 62 des Teilstücks 60' der Rohrsonde 60. Die beiden Flügelstücke 65' ,65'' sind mit der dem Führungsrohr 70 zugewandten Unterkante 67,67' mit einem Spalt 73,73' zu der ringförmigen Oberkante 72 des Führungsrohres 70 angeordnet.

In Fig.12 ist das Schneidwerkzeug 75'' in Draufsicht dargestellt und man erkennt das Führungsrohr 70, die koaxial darin angeordnete innere Rohrsonde 60 mit dem in Schlitzen 64,64' angeordneten und mit nicht dargestellten Mitteln befestigten Schneidelement 65, welches die beiden durch den Schlitz 68 teilweise voneinander getrennten Flügelstücke 65' und 65'' umfasst.

Flg.13 zeigt das in grösserem Massstab und im Schnitt dargestellte Schneidwerkzeug 75'' und man erkennt das Führungsrohr 70 sowie die darin koaxial angeordnete und mit einer Ansaugöffnung 61 versehene Rohrsonde 60 mit dem oberen, zur Befestigung des Schneidelements 65 ausgebildeten Teilstück 60'. Im dargestellten Ausführungsbeispiel ist das Schneidelement 65 mit einem im Abstand zur Innenwand 70' angeordneten Zentrierstück 74 in dem Führungsrohr 70 angeordnet.

Fig.14 zeigt im Schnitt und als Teilstück eine weitere Variante des Schneidwerkzeuges 75'' gemäss Fig.11 bis 13, wobei abweichend von dem in Verbindung mit Fig.11 bis Fig.13 beschriebenen Ausführungsbeispiel bei dieser Variante die innere Rohrsonde 60 mit einem Absatz 78 an der Innenwand 70' des Führungsrohres 70 geführt ist. Bei dieser Variante erfolgt die Infusion I' über mindestens zwei am Führungsrohr 70 vorgesehene und mit dem ringfömigen Zuführkanal 71 in Verbindung stehende Austrittsöffnungen, von welchen in Fig.14 nur die eine Austrittsöffnung 76 dargestellt ist. Die mit dem Ausführungsbeispiel gemäss Fig.11 bis 13 identischen Teile sind zur Vereinfachung mit gleicher Bezugszahl versehen.

Zur Verdeutlichung der jeweiligen Abmessung des in den Figuren 5 bis 14 dargestellten Schneidwerkzeuges 75 oder 75' beziehungsweise 75'' für die augenchirurgische Vorrichtung 100 wird an dieser Stelle darauf hingewiesen, dass der Aussendurchmesser des jeweiligen Führungsrohres 40 oder 45 beziehungsweise 70 etwa 1,5 mm und der Aussendurchmesser der inneren Rohrsonde 30 oder 50 beziehungsweise 60 etwa 1,0 mm beträgt.

Das über die Flügelstücke 35' ,35'' oder 55' ,55'' beziehungsweise 65' ,65'' des jeweiligen Schneidelements 35 oder 55 beziehungsweise 65 gemessene Mass D oder D' beziehungsweise D'', ist vorzugsweise grösser als der Durchmesser des Führungsrohres 40 oder 45 beziehungsweise 70 (Fig.6; Fig.8; Fig.12) ausgebildet. Das Mass D oder D' beziehungsweise D'' liegt etwa in der Grössenordnung von 1,6 mm. Eine Verringerung der Dimensionen D,D' oder D'' der einzelnen Teile 40;45;70 sowie 30;50;60 und 35;55;65 ist jedoch nicht ausgeschlossen.

Mit dem in die Linse 20 (Fig.1) eingeführten und rotierend sowie hochfrequent oszillierend angetriebenen Schneidelement 35 oder 55 beziehungsweise 65 der vorstehend beschriebenen Schneidwerkzeuge 75 oder 75' beziehungsweise 75'' besteht die Möglichkeit, dass die verhärtete und zu exzidierende Linsenkernstruktur in eine breiige Masse umgewandelt wird und durch Aspiration abgesaugt werden kann. Die durch die Emulgation insbesondere im bearbeiteten Linsenbereich entstehende milchige Trübung der Linse wird bei gleichzeitiger Aspiration (A,A') der breiigen Masse durch eine in den einzelnen Figuren mit I,I' bezeichnete Infusion eines geeigneten Fluids ersetzt und dadurch während des mikrochirurgischen Eingriffs am Auge eine weitgehend uneingeschränkte Sicht bis zu dem in der Linse 20 (Fig.1) befindlichen Schneidelement gewährleistet.

Durch die im wesentlichen kreisbogenförmig oder keilförmig ausgebildeten Flügelstücke 35' ,35'' oder 55',55'' beziehungsweise 65',65'' mit den daran angeordneten Schneidkanten 36,36' oder 56,56' beziehungsweise 66,66' ist beim operativen Eingriff eine Verletzung des Kapselsacks weitgehend ausgeschlossen. Die besondere Ausgestaltung des Schneidelements 35 oder 55 beziehungsweise 65 im vorderen Bereich gewährleistet, dass die während des operativen Eingriffs anfallenden Linsenteilchen beziehungsweise die breiige Linsenmasse in das Zentrum der Ansaugöffnung 31 oder 51 beziehungsweise 61 gelangen und somit abgesaugt werden können. Zusammenhängende Gewebeteilchen werden durch die Flügelstücke 35' ,35'' oder 55' ,55'' beziehungsweise 65' ,65'' zerkleinert und ebenfalls abgesaugt. Durch die in bezug auf den Aussendurchmesser des Führungsrohres 40 oder 45 beziehungsweise 70 seitlich überstehend ausgebildeten Flügelstücke 35' ,35'' oder 56' ,56'' beziehungsweise 66',66'' wird auch ein Eindringen in extrem harte Linsenkerne (Katarakts) gewährleistet.

Wie bereits erwähnt, gewährleistet das mit den Schneidelementen 35 oder 55 beziehungsweise 65 versehene Schneidwerkzeug 75 oder 75' beziehungsweise 75'', dass auch abgelöste, grössere Linsenkernstücke durch gleichzeitige Zerkleinerung sowie auch die abzulösende Zellschicht (Kortex) am Übergang vom Linsenkern zum Linsensack durch die in den einzelnen Figuren mit A und A' bezeichnete Aspiration (Absaugung) aus dem Linsensack entfernt werden können. Hierbei werden die Linsenkernstücke und die Übergangsschicht (Kortex) beziehungsweise Teilstücke derselben im Bereich der Ansaugöffnung 31 oder 51 beziehungsweise 61 gemäss Pfeilrichtung A (Fig.5; Fig.9; Fig.11) angesaugt und dabei am vorderen Teil des Schneidelements gehalten und gleichzeitig durch die rotierenden Flügelstücke zerkleinert. Die Linsenpartikel werden durch den Innenraum 30' oder 50' beziehungsweise 61 über die im Endbereich der jeweiligen inneren Rohrsonde 30 oder 50 beziehungsweise 60 vorgesehene Durchtrittsöffnung 37 oder 47 beziehungsweise 77 (Fig.4) sowie über die Saugkammer 86 und dem am Kopfstück 80 vorgesehenen Stutzen 3 in Pfeilrichtung A' abgesaugt.

Vorzugsweise wird vor dem eigentlichen operativen Eingriff, d.h. vor dem Zerkleinern des harten Linsenkerns, die nicht bezeichnete Innenseite der Hornhaut 11 mit den Endothelzellen vor der Emulgation des Linsenkerns mittels eines geeigneten, injizierten Gels benetzt. Hierdurch wird ein zuverlässiger Schutz der Zellen während der gesamten Operationsdauer erreicht, so dass Komplikationen mit abgestorbenen Endothelzellen weitgehend ausgeschlossen sind.

## Patentansprüche

1. Vorrichtung zum Zerkleinern und Entfernen des Linsenkerns aus dem Auge eines Lebewesens, bestehend aus einem im wesentlichen als Handstück ausgebildeten Gehäuse (95) und darin angeordneten Antriebsmitteln, einem an dem einen Ende des Gehäuses angeordneten Kopfstück (80) mit einem Führungsrohr (40;45;70) sowie einer koaxial darin angeordneten Rohrsonde (30;50;60), wobei das mit einem Zuführkanal versehene Führungsrohr an dem im Kopfstück angeordneten Ende mit einer Infusionsleitung verbunden und an dem anderen Ende mit mindestens einer Austrittsöffnung versehen ist und wobei die Rohrsonde an dem aus dem Führungsrohr herausragenden Ende eine Ansaugöffnung und ein um eine gemeinsame Längsachse (X) rotierend antreibbares Schneidelement (35;55;65) aufweist sowie an dem gegenüberliegend im Kopfstück angeordneten Ende mit einer Absaugöffnung versehen ist, welche zum Absaugen des überschüssigen Fluids zusammen mit den Linsenpartikeln über eine gegenüber dem Zuführkanal abgedichtete Kammer sowie über eine daran angeschlossene Saugleitung mit einer Saugpumpe verbunden ist, **dadurch gekennzeichnet,** dass die Rohrsonde (30;50;60) mit dem daran angeordneten Schneidelement (35;55;65) relativ zu dem feststehend in dem Kopfstück (80) gelagerten Führungsrohr (40;45;70) zusätzlich zu der Rotationsbewegung in axialer Richtung der Längsachse oszillierend antreibbar ist und dass das Schneidelement (35;55;65) mit mindestens zwei im westenlichen in einer die Längsachse (X) beinhaltenden Ebene liegenden Flügelstücken (35',35'';55',55'';65',65'') versehen ist.

2. Vorrichtung nach Anspruch 1, **dadurch gekennzeichnet,** dass die Rohrsonde (30;50;60) mit dem daran angeordneten Schneidelement (35;55;65) in Form einer Rotationsbewegung kombiniert mit einer hochfrequenten Oszillationsbewegung relativ zu dem Führungsrohr (40;45;70) antreibbar ist.

3. Vorrichtung nach den Ansprüchen 1 und 2, **dadurch gekennzeichnet,** dass das Schneidelement (35;55) einen in axialer Richtung von einer Ansaugöffnung (31;51) durchdrungenen sowie mit zwei diametral gegenüberliegend zueinander angeordneten Flügelstücken (35',35'';55',55'') versehenen und aus dem Führungsrohr (40;45) herausragend an der Rohrsonde (30;50) befestigten Haltekörper (34;54) aufweist.

4. Vorrichtung nach den Ansprüchen 1 bis 3, **dadurch gekennzeichnet,** dass der Haltekörper (34) im wesentlichen trichterförmig ausgebildet ist und ein an der Rohrsonde (30) befestigtes und koaxial in dem Führungsrohr (40) angeordnetes erstes zylindrisches Teilstück (33), ein konisches Teilstück (33') sowie ein zweites zylindrisches Teilstück (32) aufweist, und dass die beiden mit Schneidkanten (36,36') versehenen Flügelstücke (35' ,35'') des Schneidelements (35) in bezug auf die kreisringförmige Fläche (32') des zweiten Teilstücks (32) ebenbündig und in bezug auf die Oberkante (42) des Führungsrohres (40) mit einem Spalt (39,39') an dem Haltekörper (34) angeordnet und befestigt sind.

5. Vorrichtung nach den Ansprüchen 1 bis 3, **dadurch gekennzeichnet,** dass der Haltekörper (54) ein an der Rohrsonde (50) befestigtes und koaxial in dem Führungsrohr (45) angeordnetes zylindrisches Teilstück (54') sowie ein konisches Teilstück (53) aufweist, und dass die beiden mit Schneidkanten (56,56') versehenen Flügelstücke (55' ,55'') des Schneidelementes (55) mit geringem Abstand in bezug auf die kreisringförmige Fläche (53') des konischen Teilstücks (53) und durch einen axialen Spalt (58) beabstandete Teilstücke (57,57') im wesentlichen am konischen Teilstück (53) des Haltekörpers (54) befestigt sind.

6. Vorrichtung nach Anspruch 1, **dadurch gekennzeichnet,** dass das Schneidelement (65) durch einen in axialer Richtung der Längsachse (X) orientierten Schlitz (68) nur teilweise in zwei je mit einer Schneidkante (66,66') versehene Flügelstücke (65' ,65'') unterteilt an einem aus dem Führungsrohr (70) herausragenden Teilstück (60') der Rohrsonde (60) angeordnet und befestigt ist, wobei das herausragende Teilstück (60') der Rohrsonde (60) eine sich konisch verjüngende Mantelfläche (63) aufweist, die am vorderen Ende als kreisringförmige Schneidspitze (62) ausgebildet ist.

7. Vorrichtung nach Anspruch 6, **dadurch gekennzeichnet,** dass das Schneidelement (65) mit den Flügelstücken (65',65'') in axialer Richtung mit geringem Abstand (C) in bezug auf die kreisringförmige Schneidspitze (62) des Teilstücks (60') und mit einem Spalt (73,73') in bezug auf die Oberkante (72) des Führungsrohres (70) an der Rohrsonde (60) befestigt sowie mit einem angeformten Zentrierstück (74) versehen ist, mittels welchem das Schneidelement (65) beidseitig in geringem Abstand zur Innenwand (70') des Führungsrohres (70) angeordnet ist.

8. Vorrichtung nach Anspruch 1, **dadurch gekennzeichnet,** dass die Flügelstücke (35',35'';55',55'';65';65'') jeweils mit einer ausgehend von der dem Führungsrohr (40;45;70) zugewandten Ende sich in axialer Richtung zu der Saugöffnung (31;51; 61) konisch verjüngend ausgebildeten Schneidkante (36,36';56, 56';66, 66') versehen sind.

9. Vorrichtung nach den Ansprüchen 1 bis 8, **dadurch gekennzeichnet,** dass die mit den Schneidkanten (36,36';56,56'; 66, 66') versehenen Flügelstücke (35',35'';55',55'';65';65'') ausgehend von der Aussenkante in Richtung der Saugöffnung (31;51; 61) kreisbogenförmig oder keilförmig verjüngend ausgebildet sind.

10. Vorrichtung nach Anspruch 1, **dadurch gekennzeichnet,** dass der über die Flügelstücke (35',35'';55';55'';65';65'') des einzelnen Schneidelements (35;55;65) gemessene Abstand (D;D'; D'') grösser als der Aussendurchmesser des Führungsrohres (40; 45;70) ist, wobei der Abstand (D;D';D'') der Flügelstücke im wesentlichen in der Grössenordnung von 1,6 mm liegt.

## Claims

1. A device for pulverizing and removing the nucleus from the lens of an eye of a living creature, said device comprising a housing (95) designed substantially in the form of a handpiece with drive means arranged therein, also a nose section (80) provided with a guide tube (40;45;70) and arranged at one end of the housing, as well as a tubular probe (30;50;60) coaxially arranged in the guide tube, which is provided with a fluid supply passage and is connected to an infusion line at the end arranged in the nose section, while at the other end it is provided with at least one discharge opening, and the said tubular probe possesses at the end projecting from the guide tube an aspiration opening and a cutting element (35;55;65) that is rotatingly driven around a common longitudinal axis (X), while at the opposite end arranged in the nose section it is provided with an extraction opening which is connected to a suction pump via a chamber sealed off from the supply passage, and also via a suction line connected to said chamber, for the purpose of extracting the excess fluid together with the particles of lens material, characterized in that, in addition to executing a rotating motion, the tubular probe (30;50;60), with the cutting element (35;55;65) arranged thereon, can be oscillatingly driven along the longitudinal axis, relative to the guide tube (40;45;70) fixedly mounted in the nose section (80), and also in that the cutting element (35;55;65) is provided with at least two wing sections (35',35'';55',55''; 65' ,65'') located substantially in a plane containing the longitudinal axis (X).

2. A device according to claim 1, characterized in that the tubular probe (30;50;60) with the cutting element (35;55;65) arranged thereon is rotatingly driven in combination with a high-frequency oscillatory motion relative to the guide tube (40;45;70).

3. A device according to claims 1 and 2, characterized in that the cutting element (35;55) possesses a supporting body (34;54) attached to the tubular probe (30;50) and projecting from the guide tube (40;50), said supporting body being penetrated axially by an aspiration opening (31;51) and also being provided with two diametrically opposed wing sections (35',35'';55',55'').

4. A device according to the claims 1 to 3, characterized in that the supporting body (34) is substantially funnel-shaped and possesses a first cylindrical section (33) attached to the tubular probe (30) and coaxially arranged in the guide tube (40), a conical section (33'), as well as a second cylindrical section (32), and in that the two wing sections (35',35'') of the cutting element (35), which are provided with cutting edges (36,36'), are arranged on and attached to the supporting body (34) so that they are flush relative to the annular surface (32') of the second section (32) and so that a gap (39,39') exists relative to the upper edge (42) of the guide tube (40).

5. A device according to the claims 1 to 3, characterized in that the supporting body (54) possesses a cylindrical section (54') attached to the tubular probe (50) and coaxially arranged in the guide tube (45), and also a conical section (53), and in that the two wing sections (55',55'') of the cutting element (55), said wing sections being provided with cutting edges (56,56'), and also sections (57,57'), which are separated by an axial gap (58), are attached substantially to the conical section (53) of the supporting body (54), and the wing sections project slightly in relation to the annular surface (53') of the conical section (53).

6. A device according to claim 1, characterized in that the cutting element (65), which is only partially divided into two wing sections (65',65'') by a slot (68), said slot being axially aligned with the longitudinal axis (X) and said wing sections being provided with cutting edges (66,66'), is arranged on and attached to a section (60') of the tubular probe (60) projecting from the guide tube (70), and the projecting section (60') of the tubular probe (60) possesses a conically tapering circumferential surface (63) which at its front end is shaped to be an annular cutting tip (62).

7. A device according to claim 6, characterized in that the cutting element (65), having the wing sections (65',65''), is attached to the tubular probe (60) such that it projects axially a short distance (C) relative to the annular cutting tip (62) of section (60'), and a gap (73,73') exists relative to the upper edge (72) of the guide tube (70), and said cutting element (65) is also provided with an integrally formed centring element (74) by means of which the cutting element (65) is arranged with a small gap on each side relative to the inner wall (70') of the guide tube (70).

8. A device according to claim 1, characterized in that the wing sections (35',35'';55',55'';65';65'') are each provided with a cutting edge (36,36';56,56';66,66') which, starting from the end facing the guide tube (40;45;70), is conically tapered in axial orientation to the aspiration opening (31;51;61).

9. A device according to the claims 1 to 8, characterized in that in the direction from the outer edge towards the aspiration opening (31;51;61), the wing sections (35',35''; 55',55'';65';65'') which are provided with cutting edges (36', 36'';56',56'';66';66'') have an arcuate or wedge-shaped form.

10. A device according to claim 1, characterized in that the distance (D;D';D'') measured across the wing sections (35', 35'';55';55'';65';65'') of the individual cutting element (35; 55;65) is greater than the outer diameter of the guide tube (40;45;70), the said distance (D;D';D'') across the wing sections being substantially in the order of 1.6 mm.

## Revendications

1. Instrument pour pulvériser et enlever le noyau dur cristallin de l'oeil d'un être vivant composé d'un boîtier (95), servant pour l'essentiel à la manipulation dudit instrument, avec des dispositifs moteurs à l'intérieur, une extrémité du-dit boîtier étant équipée d'une pièce de tête (80) munie d'un tube conducteur (40;45;70) à l'intérieur duquel est disposée de façon coaxiale une sonde tubulaire (30;50;60), l'extrémité dans la pièce de tête dudit tube conducteur, qui est muni d'un canal d'alimentation, étant raccordée à un tuyau de perfusion, l'autre extrémité étant pourvue d'au moins une ouverture de sortie, ladite sonde tubulaire étant équipée, à l'extrémité dépassant le tube conducteur, d'une ouverture d'aspiration et d'un élément coupant (35;55;65) pouvant être actionné par un mouvement de rotation autour d'un axe longitudinal (X) commun, au sortir de l'autre extrémité de ladite sonde tubulaire, située dans la pièce de tête, le liquide excédentaire et les particules du cristallin étant aspirés vers une chambre étanche par rapport au canal d'alimentation, ladite chambre étant elle-même raccordée par un tuyau d'aspiration à une pompe d'aspiration, caractérisé en ce que la sonde tubulaire (30;50;60) munie de l'élément coupant (35;55;65) peut être actionnée, par rapport au tube conducteur (40;45;70) disposé de façon fixe dans la pièce de tête (80), en plus du mouvement rotatif par un mouvement oscillatoire en direction axiale de l'axe longitudinal, et en ce que l'élément coupant (35;55;65) est équipé d'au moins deux pièces à ailettes (35',35'';55',55'';65',65'') se trouvant pour l'essentiel sur un plan contenant l'axe longitudinal (X).

2. Instrument selon la revendication 1, caractérisé en ce que la sonde tubulaire (30;50;60) munie de l'élément coupant (35;55;65) peut être actionnée, par rapport au tube conducteur (40;45;70), de façon combinée, par un mouvement de rotation et par un mouvement d'oscillation à haute fréquence. teur (40;45;70), de façon combinée, par un mouvement de rotation et par un mouvement d'oscillation à haute fréquence.

3. Instrument selon les revendications 1 et 2, caractérisé en ce que l'élément coupant (35;55) présente une pièce de blocage (34;54), fixée à la sonde tubulaire (30;50) et saillissant du tube conducteur (40;45); ladite pièce de blocage étant pénétrée en direction axiale par une ouverture d'aspiration (31;51) et munie de deux pièces à ailettes (35',35'';55',55''), disposées de façon diamétralement opposée.

4. Instrument selon les revendications 1 à 3, caractérisé en ce que la pièce de blocage (34) est pour l'essentiel en forme d'entonnoir et qu'elle présente une première partie cylindrique (33), fixée à la sonde tubulaire (30) et disposée de façon coaxiale dans le tube conducteur (40), puis une partie conique (33') ainsi qu'une deuxième partie cylindrique (32), et que les deux pièces à ailettes (35',35'') de l'élément coupant (35), munies d'arêtes coupantes (36,36'), sont disposées et fixées à la pièce de blocage (34), de façon plane par rapport à la surface en anneau de cercle (32') de la deuxième partie (32), et avec un petit espace (39,39') par rapport au bord supérieur (42) du tube conducteur (40).

5. Instrument selon les revendications 1 à 3, caractérisé en ce que la pièce de blocage (54) présente une partie cylindrique (54'), fixée à la sonde tubulaire (50) et disposée de façon coaxiale dans le tube conducteur (45) ainsi qu'une partie conique (53), et que les deux pièces à ailettes (55',55'') de l'élément coupant (55), munies d'arêtes coupantes (56,56'), sont fixées pour l'essentiel, avec un petit espace par rapport à la surface en anneau de cercle (53') de la partie conique (53), et par l'intermédiaire des parties (57,57'), séparées par une fente axiale (58), sur la partie conique (53) de la pièce de blocage (54).

6. Instrument selon la revendication 1, caractérisé en ce que l'élément coupant (65) est fixé sur une partie (60') de la sonde tubulaire (60), saillissant du tube conducteur (70), et disposé de façon partiellement divisée, par une fente (68) orientée en direction axiale de l'axe longitudinal (X), en deux pièces à ailettes (65',65''), chacune étant pourvue d'une arête coupante (66,66'), la partie saillissante (60') de la sonde tubulaire (60) présentant une surface latérale effilée en cône (63), constituant à l'extrémité antérieure une lame coupante en forme d'anneau de cercle (62).

7. Instrument selon la revendication 6, caractérisé en ce que l'élément coupant (65) avec les pièces à ailettes (65',65'') est fixé à la sonde tubulaire (60), en direction axiale avec une petite distance (C) par rapport à la lame coupante en forme d'anneau de cercle (62) de la partie (60'), et avec un petit espace (73,73') par rapport au bord supérieur (72) du tube conducteur (70), ledit élément coupant (65) étant muni d'une pièce de centrage (74) grâce à laquelle l'élément coupant (65) est disposé des deux côtés, à petite distance par rapport à la paroi interne (70') du tube conducteur (70).

8. Instrument selon la revendication 1, caractérisé en ce que les pièces à ailettes (35',35'';55',55'';65',65'') sont, chacune, pourvues d'une arête coupante (36,36';56,56';66,66') qui est, en partant de l'extrémité tournée vers le tube conducteur (40;45;70) et en allant en direction axiale vers l'ouverture d'aspiration (31;51;61), effilée en cône.

9. Instrument selon les revendications 1 à 8, caractérisé en ce que les pièces à ailettes (35',35'';55',55'';65',65'') munies d'arêtes coupantes (36,36';56,56';66,66') sont, en partant du bord extérieur et en allant vers l'ouverture d'aspiration (31;51;61), effilées en coin ou en arc de cercle.

10. Dispositif selon la revendication 1, caractérisé en ce que la dimension (D;D';D''), mesurée au-dessus des pièces à ailettes (35',35'';55';55'';65';65'') de chaque élément coupant (35;55;65), est supérieure au diamètre extérieur du tube conducteur (40;45;70), la dimension (D;D';D'') des pièces à ailettes étant pour l'essentiel de l'ordre de 1,6 mm.
